Europäisches Patentamt

European Patent Office

Office européen des brevets

(19)

(11) Publication number : **0 417 146 B1**

## EUROPEAN PATENT SPECIFICATION

(12)

(45) Date of publication of patent specification :
**04.11.92 Bulletin 92/45**

(51) Int. Cl.⁵ : **A61F 5/00**, **E03D 11/00**,
**A47K 13/24**

(21) Application number : **89906013.1**

(22) Date of filing : **31.05.89**

(86) International application number :
**PCT/BE89/00024**

(87) International publication number :
**WO 89/11838 14.12.89 Gazette 89/29**

(54) **DEVICE FOR THE FACILITATION OF DEFECATION.**

(30) Priority : **03.06.88 BE 8800632**

(43) Date of publication of application :
**20.03.91 Bulletin 91/12**

(45) Publication of the grant of the patent :
**04.11.92 Bulletin 92/45**

(84) Designated Contracting States :
**AT BE CH DE FR GB IT LI LU NL SE**

(56) References cited :
**EP-A- 0 218 122**
**FR-A- 2 601 585**
**US-A- 3 004 534**
**US-A- 3 550 586**

(73) Proprietor : **CLOOSTERMANS-HUWAERT,
Cecile, Pierre, Marie, Yvonne
Waterstraat 13
B-9308 Gijzegem (BE)**

(72) Inventor : **CLOOSTERMANS-HUWAERT,
Cecile, Pierre, Marie, Yvonne
Waterstraat 13
B-9308 Gijzegem (BE)**

(74) Representative : **Debrabandere, René
BUREAU DE RYCKER Vereenigde
Octrooibureaux Belgie N.V. Arenbergstraat 13
B-2000 Antwerpen (BE)**

Jouve, 18, rue Saint-Denis, 75001 PARIS

## Description

The invention relates to a device for the facilitation of defecation.

Constipation can be caused by a slow advancement of the faeces, whereby many factors, such as intestinal locomotion, the fecal material, intestinal flora, hormonal factors etc. play a role. Yet it is also known that with some people constipation is caused by a difficulty to evacuate the faeces. The increase in abdominal pressure, the coordinated working of reflexes and muscles, the form and proportions of the anorectal junction and anal tract play a role with this, and often this form of constipation is coupled with an over large displacement, mainly descent, of the pelvic floor, by which It is thought that this is a consequence of evacuation difficulty and pressing hard.

The most classic approach to constipation Is the use of fibre rich food and laxatives; these have, however, no direct influence on the evacuation mechanism itself and do not prevent the descent.

Furthermore some people also assume mechanical and physical methods, which by means of pressure, massage and vibrations process the fecal matter In the rectum and/or Induce reflexes for defecation. These are, therefore, active radical methodes or apparatus, which already intervene before the expulsion begins, that also influence the normal defecation process. Possibly these have some use when the reflexes are disturbed and with over large fecal matter. These also offer no solution to specifically limit the exaggerated displacement of the pelvic floor, where required.

US-A-3 004 534 being the most pertinent document (preamble of claim 1) discloses such active apparatus to be mounted on a toilet and comprising a support for the perineal region of the pelvic floor. Said support consists of two massaging or manupulating members securely affixed to shafts which can be rotated by the user operating a crank. Both members extend longitudinally, on both sides of the middle of the pelvic floor. Said apparatus acts by pressing and massaging and in fact the members transmit a massaging action to the buttocks and the rectal regions of the patient.

An exaggerated descent of the pelvic floor during the pressing is not limited by the members from the above mentioned apparatus according to US-A-3 004 534. Descent of the pelvic floor between said members is possible.

Sometimes an operation, consisting of a lifting, is suggested, yet this is a major decision, of which the result cannot always be predicted.

Through use of the device according to the invention in question another way of thinking has arisen, whereby the descent appears to be not only the result but a cause of constipation. Thus a following plan is assumed for what regards this form of constipation.

A good resistance of the pelvic floor is important for the normal course of the evacuation of the faeces during defecation. During defecation there is normally a slight descent of the pelvic floor. With some people, mainly women, there is a decreased resistance during the defecation and an over large displacement of the pelvic floor occurs, mainly an exaggerated descent, either coupled or not with a forward component. Through the exaggerated descent the bowel movements are impeded and an obstructive form of constipation occurs. A viscious circle arises: the more descent, the more constipation and therefore need to press harder, which in turn leads to further descent. At a later stage additional incontinence can even occur through nerve damage as a result of stretching of this nerve.

The present invention is intended as a device or remedy to provide for the facilitation of defecation in this case of constipation, which are caused by an over large displacement, mainly descent of the pelvic floor which occurs at the moment of squeezing and straining and therefore during the bowel motions and this without impeding the normal physiological process of slight descent and opening of the anorectal corner. This device can also possibly be useful as preventative remedy. For this purpose the invention comprises a support for the perineal region of the pelvic floor of a person between the anal and urogenital region, to limit a pathological displacement, mainly descent of the pelvic floor, when pressing.

This support is situated there where the pressure is greatest and the resistance of the pelvic floor is weakest, this is thus on the central region at the height of the perineum. In order not to impede the normal slight physiological descent of the pelvic floor which occurs during the defecation, restriction happens at the level under the surface of the top of the WC seat. It is, therefore, not the intention to exert an active pressure but only to provide a passive support of the pelvic floor at the moment of pressing during defecation, as soon as the movement of the pelvic floor has become too great.

This pathological displacement is usually mainly an exaggerated descent, but can also include a forward component which can possibly be held by a perpendicular surface on this axle of displacement. For these reasons in a special embodiment the support can be provided with an inclination. The support can be directed from the front down towards the back so that when pressing the pelvic floor is also supported in front.

The support of which the width is obviously changeable, can furthermore be adjusted to the perineal region of the pelvic floor of the user and so can possibly be provided with a raised piece.

In a note-worthy embodiment of the invention the support is mounted on a toilet with a folding down seat and situated under the top of the seat. The support can either be part of a toilet bowl, or a toilet seat, or even an accessory which is destined to be placed on a toilet.

From examination of other of the above mentioned known devices and accessories based on physical and mechanical methods it is found that these essentially differ from the device according to the invention.

According to FR-A-2 601 585 a device which includes projections to actively exert a pressure on the perineal zone of the pelvic floor, is mounted on a toilet. These projections are normally situated at the back of the anus but with an embodiment the projections are spread around the anus. These projections consequently ensure an active exertion of pressure and massage with the aim to allow the person to move on these projections which are found above the surface of the toilet seat to so induce the reflexes in order to start the bowel motions. This device appears irritating in use and in many pathological cases may not be applied due to danger of injuries, considering the active manipulating effect.

From US-A-2 985 171 it is known that an accessory "colon manipulator", to be mounted behind the seat of a toilet, consists of an arm which bears an upward protruding finger on its extremity. This finger exerts a local pressure on the pelvic floor of the user, just at the rear of the anus. Also here an active pressure on the rectum is exerted and there is no transversal connection provided between the anal and urogenital region, so that at that place at the height of the perineum no support occurs. This device appears rather inconvenient and does not prevent the pathological displacement in a passive manner and in the place where it is necessary, mainly descent with or without being coupled with a forward component.

From EP-A-O 218 122 a support is known for the pelvic floor musculature. This support, however, only supports the pelvic floor between the anus and the coccyx. In some cases a vibrator is also mounted in the support to induce the defecation. There is no transversal connection provided between the urogenital region and anal region, so that as formulated with US-A-2 985 171 the pathological displacement is not prevented where necessary. Support behind the anus does not prevent the descent and the turning over of the pelvic floor at the height of the perineum, and appears to us rather inconvenient for the normal opening of the anorectal corner.

Other details and advantages of the invention will appear from the following description of a device for the facilitation of defecation, according to the invention; this description is only given as an example and does not restrict the invention; the reference numbers relate to the enclosed drawings.

Figure 1 is a top view of the device for the facilitation of the defecation according to the invention, which is part of a toilet bowl;

figure 2 presents a cross-section according to the line II-II from figure 1;

figure 3 is a top view of a device according to the invention analogue to that from figure 1 but whereby the device is produced as a separate piece and is mounted on the toilet;

figure 4 presents a cross-section according to the line IV-IV from figure 3;

figure 5 is a top view analogue to that from figure 3 but relating to another embodiment of the device;

figure 6 presents a cross-section according to the line VI-VI from figure 5 whereby the seat is drawn in dashed line;

figure 7 is a top view analogue to that from the figures 3 and 5, but relating to yet another embodiment of the invention;

figure 8 presents a cross-section according to the line VIII-VIII from figure 7;

figure 9 is a front view of the device according to the invention relating to yet another embodiment of the invention, whereby a seat of a toilet is represented in a dashed line;

figure 10 is a top view of the device from figure 9, also with the seat in dashed line;

figure 11 is a front view analogue to thai from figure 9 of a device according to the invention, but relating to yet another embodiment and also with the seat and a part of the toilet bowl represented in dashed line;

figure 12 is a top view of the device from figure 11, with the seat of the toilet in dashed line.

In the different figures the same reference numbers relate to the same elements.

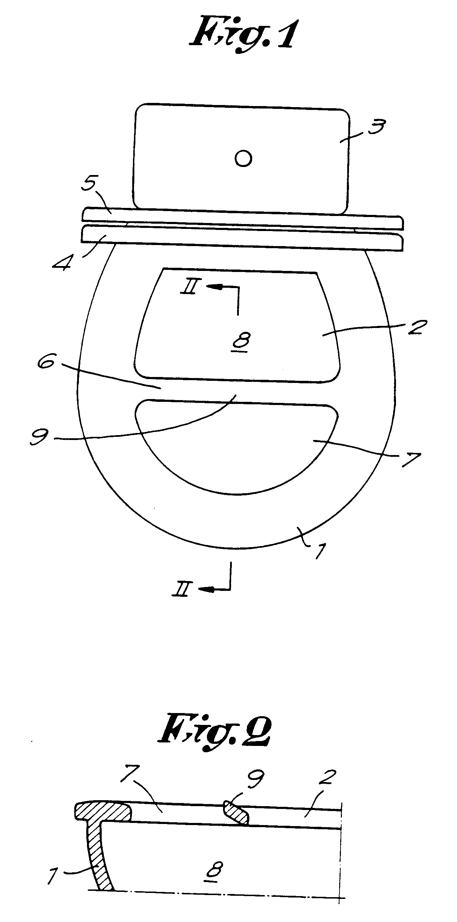

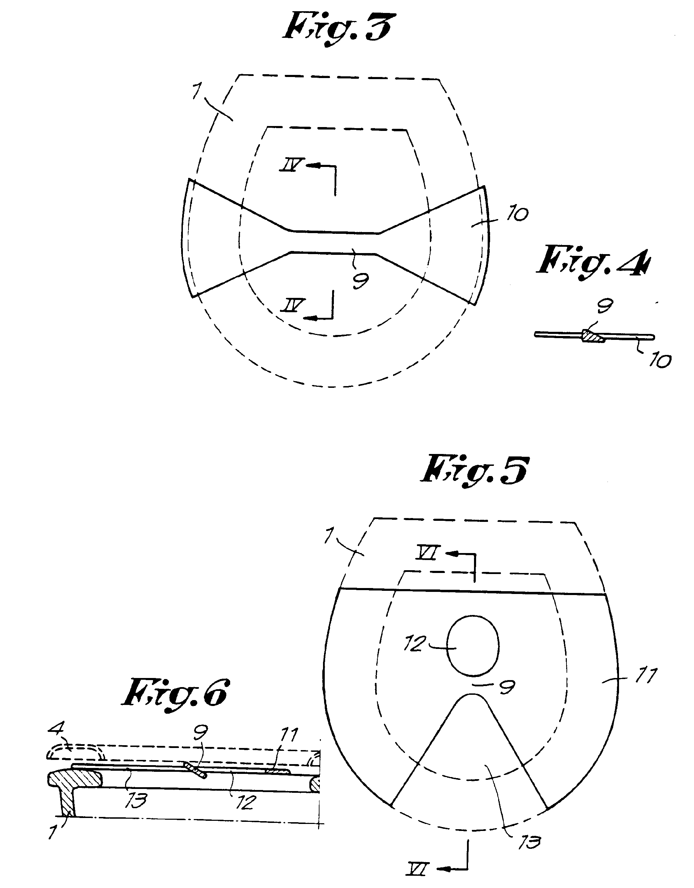

The toilet according to figures 1 and 2 includes in the usual manner a toilet bowl 1 of glazed porcelain which is provided with a funnel-shaped opening 2 facing upwards.

Above this bowl 1, behind the opening 2, a flush cistern 3 is mounted which can be connected in the usual manner with the inside of the bowl 1 and is connected to a water pipe not shown in the figures for the sake of simplicity. The bowl 1 is also connected in the usual manner to a drain pipe not shown in the figure.

In front of the flush cistern 3, a folding down seat 4 is also mounted on top of the bowl 1, while a folding down lid 5 is mounted on top of the seat 4.

Characteristic of the invention is the fact that on top of the opening 2, a transversal connection 6 is mounted which divides the opening 2 on top into a front opening 7 and a back opening 8.

The middle port of the transversal connection 6 is provided with a raised piece on top of which the back, this is directed to the flush cistern 3 side, extends down towards the back. The top of the raised piece is situated a little under the top of the seat 4 when this is folded down on the bowl 1. This middle part of the transversal connection 6 forms thus a support 9 for the perineal region of the pelvic floor of the user, which support does not actively press on the user who is sitting on the seat 4 but only prevents an over large descent of the pelvic floor of this person while pressing during defecation. The normal descent of the pelvic floor which appears during this pressing, is therefore not impeded but only an exaggerated descent, which hampers the bowel motions, is impeded.

With exaggerated descent the pelvic floor is supported in front and underneath.

For healthy people, who show no exaggerated descent during pressing, the support can work preventively and counteract the development of an exaggerated descent.

In any case the toilet remains normally serviceable for such healthy people.

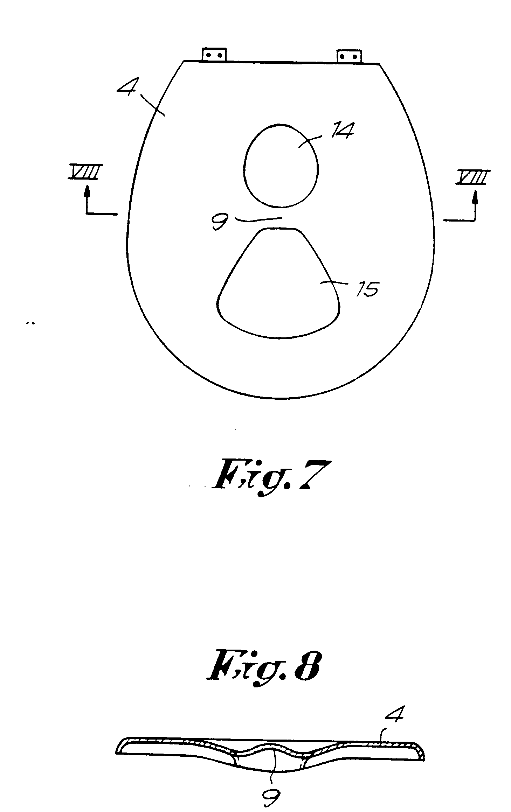

The device for limiting the descent during pressing need not necessarily form part of the whole of the bowl 1. In place of a transversal connection 6 of the bowl 1 this device with the support 9 can be a separate accessory as represented in the figures 3 through 12.

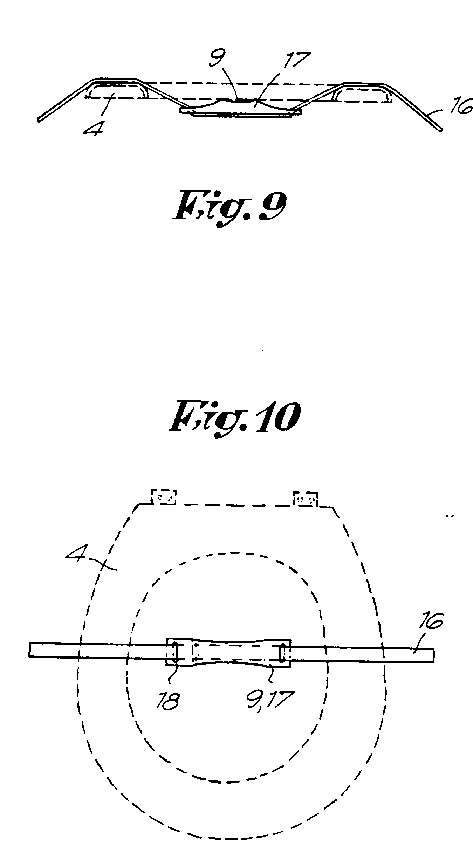

The accessory according to figures 3 and 4 is a strip 10 with widening extremities of which the outer edges are folded down.

The middle port of the strip 10 forms the support 9. The strip 10 is placed in a transversal direction on the bowl 1 whereby the folded extremities hook over opposite locations on the upper edge of the bowl. Thereby sufficient of the opening 2 remains free behind the strip 10 for defecation while sufficient of the opening 2 remains open in front of the strip 10 for urination.

The middle part of the strip 10 which forms the support 9, is equipped with a raised piece that is fitted to the part of the Pelvic floor of the user which is situated between the anal region and the uro-genital region. At the back this raised piece extends out slanting from top to bottom by which the top of the raised piece is situated slightly under the folded down seat. It is, however, not necessary that the seat be folded down during the defecation. If necessary, the strip 10 can easily be removed, either for cleaning, or for use of the toilet by users who do not need to use the device.

The strip 10 may be made of any material, such as metal or plastic.

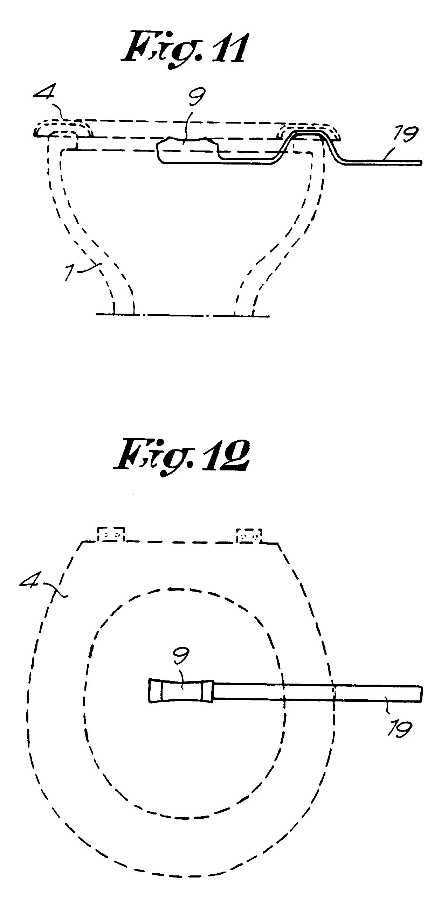

In the embodiment according to figures 5 and 6 the accessory is equipped with a plate with two spaces, namely are almost round opening 12 behind for defecation and an almost trapezium-shape cutting out 13 in front for urination.

The middle part of the plate 11 which is situated between the opening 12 and the cutting out 13, forms the support 9 and is equipped with a similar raised piece as the middle part of the strip 10 in the embodiment according to figures 3 and 4.

The plate 11 is placed on the bowl 1 under the seat 4, obviously with the opening 12 behind.

The embodiment of the device according to the figures 7 and 8 differs from the embodiment according to the figures 5 and 6 in that the support 9 is not part of a plate 11 which is placed between the bowl 1 and the seat 4 but forms part of the seat 4 itself which is not equipped as usual with a central opening but does have the form of a plate with two openings 14 and 15 situated one behind the other which correspond with the above mentioned opening 12 and cutting out 13.

The opening 14 behind is almost round, white the front opening 15 is almost trapezium-shaped.

For sturdiness these openings 14 and 15 are surrounded by folded down edges, as is the outer edge of the seat 4 also folded down.

The part of the seat situated almost in the middle of the openings 14 and 15 forms the above mentioned support 9 for the perineal region of the pelvic floor during the pressing. It is, therefore, also equipped as in the former embodiment with a raised piece that is fitted to the perineal region of the pelvic floor and is situated slightly lower than the parts of the seat 4 situated more towards the outside.

The figures 9 and 10 relate to an embodiment of the device whereby the support 9 again forms part of an accessory which is loose from the rest of the toilet.

The accessory represented in these figures 9 and 10 consists of a souple band 16 on which an elongated folded small plate 17 is slid in the middle.

The band 16 extends through two small slots 18 situated near the extremities of the small plate 17 so that the band 16 is situated underneath between the small slots 18 and on the top of the outside of the small slots 18.

The accessory 16, 17 is so placed that the band 16 extends transversally over the folded down seat 4 on the bowl 1 and the little plate 17 situated almost in the middle above the opening 2, so that a person who is seated on the seat 4, has the perineal region of his pelvic floor opposite the small plate 17 which forms a raised piece.

During the pressing the person who is seated on the seat 4 should hold the extremities of the band 16 on both sides of the toilet and possibly push these down so that an exaggerated descent of the pelvic floor is limited during this pressing.

Through the fact that the small plate 17 is fixed on a band 16, the raised piece of this small plate 17 can easily fit to the shape of the pelvic floor so that strictly speaking this raised piece need not necessarily be directed behind from above to below. The accessory 16, 17 can after use be folded up into a small pack and easily be taken along.

In a variant of this embodiment the band does not run through on the small plate 17 but has the accessory two band parts between which the small plate 17 is fixed.

In order to avoid that one has to hold the extremities of the band 16, means can be applied in a variant to fix these extremities to the toilet and moreover to the seat 4 of it. On the extremities a clasp may be mounted, for example, so that these extremities can be stretched around parts of the seat 4. The seat 4 or the bowl 1 can also be equipped.with hooks or other means of attachment on which the extremities of the band 16 may be secured.

In the embodiment according to the figures 11 and 12 the device consists of a lever 19 that has a head at one extremity which is placed on top of the above mentioned raised piece and forms the support 9.

Between both extremities the lever 19 is equipped with a U-shaped fold facing upwards with which it can be placed over the upper edge of the toilet bowl 1, under the seat 1 as is clearly visible in figure 11.

The distance between these folds and the support 9 is such that this support is situated almost in the middle of the opening, opposite the perineal region of the pelvic floor of a person who is seated on the folded down seat.

Also here this person should hold the extremity of the lever 19 and if necessary push downwards with the necessary force to limit the descent of the pelvic floor during the pressing.

Also with this embodiment the device can easily be taken along.

If necessary a folding lever 9 can be manufactured.

In all embodiments the addition of the device is relatively inexpensive.

In all embodiments except the first whereby the support is permanently fixed to the toilet bowl, the device can easily be installed on an existing toilet. The device can as such be put on the market whereby the raised piece of the support 9 can be adapted separately for each user if necessary. Since the device is relatively inexpensive, it can also easily be replaced.

In the embodiments by which The support 9 is neither part of the toilet bowl 1 nor of the seat 4 or the lid 5, the device with the support 9 may easily be removed, for example to be cleaned or to be taken along to be placed on another existing toilet.

The invention is in no way restricted to the embodiments described for this purpose, and in the scope of the claims many changes may be applied to the embodiments described, among others to the shape, the construction, the arrangement and the number of the ports which are used for the realisation of the invention. Especially the shape of the accessories are only given as examples.

Also the shape of the spaces or cutouts are only given as examples. Apart from that the location and the shape of these spaces and cutouts differ in many case according to the user.

## Claims

1. Device for the facilitation of defecation, comprising a support (9) being situated under the upper surface of a toilet seat for the perineal region of the pelvic floor of a person, characterised in that said support (9) lies transversally between the anal region and the uro-genital region, to limit a pathological displacement, mainly descent of the pelvic floor, during the pressing.

2. Device according to claim 1 characterised in that the perineal support (9) is provided with an inclination.

3. Device according the claim 2, characterised in that the perineal support (9) shows an inclination which is directed downwards from front to back so that also a forward movement of the pelvic floor is limited.

4. Device according to one of the claims 1 through 3, characterised in that the perineal support (9) is provided with a raised piece and is adapted to the shape of the perineal region of the pelvic floor of the user.

5. Device according to one of the claims 1 through 4, characterised in that the support (9) is mounted on a toilet (1-4) with folding down seat (4) and is situated under the upper surface of the seat (4).

6. Device according to one of the claims 1 through 5, characterised in that the support (9) is partt of a toilet bowl (1).

7. Device according to claim 6, characterised in that the support (9) is part of a transversal connection (6) which divides the opening (2) on the top of the toilet bowl (1) into a front opening (7) and a back opening (8).

8. Device according to one of the claims 1 through 6, characterised in that the support (9) is part of a toilet seat (4).

9. Device according to claim 8, characterised in that the support (9) is formed by the part of the toilet seat (4) between a front space (15) and a back space (14).

10. Device according to one of the claims 1 through 6, characterised in that the support (9) is port of an accessory (10, 11, 16-17 or 19) which is destined to be placed on a toilet.

11. Device according to claim 10, characterised in that the accessory is a plate (11) which has a space (12) at the back for defecation and a space (13) in front for urination, by which the support (9) is formed between both spaces (12, 13).

12. Device according to claim 10, characterised in that the accessory is a strip (10) destined to be placed transversally over a toilet bowl (1) so that an opening in front of the strip for urination and an opening behind the strip for defecation remain open, of which strip (10) the middle part forms the support.

13. Device according to claim 10, characterised in that the accessory (16, 17) contains a souple band destined to be stretched over the opening (12) of a toilet bowl (1), and a relatively rigid part (17) which forms the support (9) and is mounted on the souple band (16) or between parts thereof.

14. Device according to claim 10, characterised in that the accessory is a lever (19) which is destined to rest between its extremities on the edge of a toilet bowl (1) with one extremity in or opposite the opening (2) of the toilet bowl (1), on which extremity the support (9) is mounted.

**Patentansprüche**

1. Gerät zur Vereinfachung der Defäkation, bestehend aus einer Unterstützung (9) der Perinealregion des Beckenbodens, die sich unterhalb der Oberfläche der Brille einer Toilette befindet, dadurch gekennzeichnet daß die genannte Unterstützung (9) quer zwischen dem Analgebiet und der Uro-Genitalgegend liegt mit dem Zweck der Einschränkung einer pathologisch bedingten Verlagerung, hauptsächlich Senkung des Beckenbodems, während des Pressens.

2. Gerät nach Anspruch 1 dadurch gekennzeichnet daß die perineale Unterstützung (9) versehen ist mit einer Abschrägung.

3. Gerät nach Anspruch 2, dadurch gekennzeichnet daß die perineale Unterstützung (9) eine Abschrägung aufweist die von vorn bis hinten nach unten gerichtet ist sodaß also auch eine Vorwärtsbewegung des Beckenbodens beschränkt wird.

4. Gerät nach einem der Ansprüche 1 bis 3, dadurch gekennzeichnet daß die perineale Unterstützung (9) versehen ist mit einem erhabenen Teil und der Form der Perinealregion des Beckenbodens des Benutzers angepasst worden ist.

5. Gerät nach einem der Ansprüche 1 bis 4, dadurch gekennzeichnet daß die Unterstützung (9) auf eine Toilette montiert ist mit einer Klappbrille (4) und sich unterhalb des oberen Flaches der Brille (4) befindet.

6. Gerät nach einem der Ansprüche 1 bis 5, dadurch gekennzeichnet daß die Unterstützung (9) Teil eines Klosettbeckens (1) ist.

7. Gerät nach Anspruch 6, dadurch gekennzeichnet daß die Unterstützung (9) Teil einer Querverbindung (6) ist die die Offnung (2) am oberen Ende des Klosettbeckens (1) zerteilt in eine vordere Offnung (7) und eine hintere Offnung (8).

8. Gerät nach einem der Ansprüche 1 bis 6, dadurch gekennzeichnet daß die Unterstützung (9) Teil ist einer Klosettbrille (4).

9. Gerät nach Anspruch 8, dadurch gekennzeichnet daß die Unterstützung (9) gebildet wird von dem Teil der Klosettbrille (4) zwischen einem vorderen Raum (15) und einem hinteren Raum (14).

10. Gerät nach einem der Ansprüche 1 bis 6, dadurch gekennzeichnet daß die Unterstützung (9) Teil ist einer Hilfsvorrichtung (10, 11, 16-17 oder 19) die dazu bestimmt ist auf eine Toilette gesetzt zu werden.

11. Gerät nach Anspruch 10, dadurch gekennzeichnet daß die Hilfsvorrichtung eine Platte (11) ist die an der Hinterseite einen Raum (12) hat für Defäkation und einen Raum (13) vorn für Urinieren, wobei die Unterstützung (9) gebildet wird zwischen beiden Räumen (12, 13).

12. Gerät nach Anspruch 10, dadurch gekennzeichnet daß die Hilfsvorrichtung ein Streifen (10) ist, dazu bestimmt quer über ein Klosettbecken (1) angeordnet zu werden sodaß vor dem Streifen eine Offnung bleibt zum Urinieren und hinten eine Offnung zur Defäkation, wobei der mittlere Teil des Streifens (10) die Unterstützung bildet.

13. Gerät nach Anspruch 10, dadurch gekennzeichnet daß die Hilfsvorrichtung ((16, 17) ein geschmeidiges Band besitzt dazu bestimmt um über die Offnung (12) eines Klosettbeckens (1) gespannt zu werden, und einen relativ steifen Teil (17) der die Unterstützung (9) bildet und auf das geschmeidige Band (17) montiert worden ist oder zwischen Teile dessen.

14. Gerät nach Anspruch 10, dadurch gekennzeichnet daß die Hilfsvorrichtung ein Hebel (19) ist dazu bestimmt zwischen seinen Enden auf der Kante eines Klosettbeckens (1) zu ruhen mit einem Ende in oder gegenüber der Offnung (2) des Klosettbeckens (1), auf dessen Ende die Unterstützung (9) montiert worden ist.

**Revendications**

1. Dispositif pour faciliter la défécation, comprenant un support (9) situé en-dessous de la surface d'une lunette de W.C. destiné à la région périnéale du plancher pelvien d'une personne, caractérisé en ce que ledit support (9) est disposé transversalement entre la région anale et la région urogénitale, dans le but de limiter un déplacement pathologique, principalement la descente du plancher pelvien, lorsque s'exerce la pression.

2. Dispositif selon la revendication 1, caractérisé en ce que le support périnéal (9) est muni d'une inclinaison.

3. Dispositif selon la revendication 2, caractérisé en ce que le support périnéal (9) présente une inclinaison qui est orientée vers le bas d'avant en arrière, de telle sorte qu'on limite également un mouvement du plancher pelvien vers l'avant.

4. Dispositif selon une des revendications 1 à 3, caractérisé en ce que le support périnéal (9) est équipé d'un élément faisant saillie, et est adapté à la forme de la région périnéale du plancher pelvien de l'utilisateur.

5. Dispositif selon une des revendications 1 à 4, caractérisé en ce que le support périnéal (9) est monté sur un W.C (1-4) à siège abattant (4) et est situé en-dessous de la surface supérieure du siège (4).

6. Dispositif selon une des revendications 1 à 5, caractérisé en ce que le support (9) fait partie d'une cuvette (1) de W.C.

7. Dispositif selon la revendication 6, caractérisé en ce que le support (9) fait partie d'un raccord transversal (6) qui subdivise l'ouverture (2) au sommet de la cuvette (1) du W.C. en une ouverture antérieure (7) et en une ouverture postérieure (8).

8. Dispositif selon une des revendications 1 à 6, caractérisé en ce que le support (9) fait partie d'un siège (4) de W.C.

9. Dispositif selon la revendication 8, caractérisé en ce que le support (9) est formé par la partie du siège de W.C. (4) s'étendant entre un espace antérieur (15) et un espace postérieur (14).

10. Dispositif selon une des revendications 1 à 6, caractérisé en ce que le support (9) fait partie d'un accessoire (10, 11, 16-17 ou 19) qui est destiné à être placé sur un W.C.

11. Dispositif selon la revendication 10, caractérisé en ce que l'accessoire est une plaque (11) munie d'un espace (12) à l'arrière pour la défécation et d'un espace (13) à l'avant pour la miction, par lequel le support (9) est formé entre les deux espaces (12, 13).

12. Dispositif selon la revendication 10, caractérisé en ce que l'accessoire est une bande (10) destinée à être placée transversalement par dessus une cuvette de W.C. (1), de telle sorte qu'une ouverture à l'avant de la bande, destinée à la miction, et qu'une ouverture à l'arrière de la bande, destinée à la défécation, restent ouvertes, la partie médiane de la bande (10) formant le support.

13. Dispositif selon la revendication 10, caractérisé en ce que l'accessoire (16, 17) contient une bande souple destinée à être étirée par-dessus l'ouverture (12) d'une cuvette de W.C (1), ainsi qu'une partie relativement rigide (17) qui forme le support (9), et est monté sur la bande souple (16) ou entre les parties de cette dernière.

14. Dispositif selon la revendication 10, caractérisé en ce que l'accessoire est un levier (19) qui est destiné à venir s'appuyer entre ses extrémités sur le bord d'une cuvette de W.C. (1), une extrémité sur laquelle est monté le support (9) étant située dans ou face à l'ouverture (2) de la cuvette de W.C. (1).

# *Fig.1*

# *Fig.2*

Fig.3

Fig.4

Fig.5

Fig.6

*Fig.7*

*Fig.8*

## Fig. 9

## Fig. 10

# Fig. 11

# Fig. 12